## Europäisches Patentamt

## European Patent Office

(19) 

## Office européen des brevets

(11) Publication number: **0 040 830**
**B1**

(12) **EUROPEAN PATENT SPECIFICATION**

(45) Date of publication of patent specification: **21.09.83**

(51) Int. Cl.³: **C 07 C 47/24, C 07 C 45/58**

(21) Application number: **81103927.0**

(22) Date of filing: **21.05.81**

(54) Method for the preparation of (E)-4-bromo-2-methylbut-2-en-1-al.

(30) Priority: **23.05.80 US 152700**

(43) Date of publication of application:
**02.12.81 Bulletin 81/48**

(45) Publication of the grant of the patent:
**21.09.83 Bulletin 83/38**

(84) Designated Contracting States:
**BE CH DE FR GB IT LI NL**

(56) References cited:
**GB - A - 1 037 751**
**US - A - 4 054 608**

(73) Proprietor: **J.T. Baker Chemical Co.**
**222 Red School Lane**
**Phillipsburg, New Jersey 08865 (US)**

(72) Inventor: **Gray, Gary M.**
**1572 Ralston Road**
**Betlehem Pennsylvania (US)**

(74) Representative: **Vossius, Dorothea**
**VOSSIUS VOSSIUS TAUCHNER HEUNEMANN**
**RAUH P.O. Box 86 07 67 Siebertstrasse 4**
**D-8000 Munich 86 (DE)**

Courier Press, Leamington Spa, England.

Method for the preparation of (E)-4-bromo-2- methylbut-2-en-1-al

This invention relates to a method for the preparation of (E)-4-bromo-2-methylbut-2-en-1-al by brominating isoprene epoxide with cupric bromide in the presence of lithium carbonate.

The compound (E)-4-bromo-2-methylbut-2-en-1-al is known in the art and is important for its use as a starting material for (E)-4-acetoxy-2-methylbut-2-en-1-al a key intermediate in one of the industrial synthesis of Vitamin A and Vitamin A acetate. Moreover, the compound can also be used as an intermediate in the synthesis of various other boilogically active compounds containing isoprene derived groups.

It has heretofore been proposed by G. Eletti—Bianchi et al., J. Org. Chem., 41, 1648—50 (1976) to prepare the corresponding chlorinated derivative of isoprene epoxide by chlorinating isoprene epoxide with cupric chloride in the presence of lithium chloride as a catalyst. A similar disclosure is to be found in U.S. Patent 4,054,608 issued October 18, 1977, to Luciano Re et al., and assigned to Anic S.p.A.

In the article by G. Eletti—Bianchi et al. the authors indicate that the synthesis of (E)-4-chloro-2-methylbut-2-en-1-al from 3,4-epoxy-3-methyl-1-butene and copper chloride is accomplished through a mechanism wherein there is a nucleophilic attack of a chloride ion on the terminal olefinic carbon in 3,4-epoxy-3-methyl-1-butene. However, since a bromide ion is a much poorer nucleophile than the chloride ion, copper bromide would not be expected to be sufficiently reactive to be utilized in such a process. Moreover, since the reaction produces a hydrohalic acid and the desired product of the present application, namely (E)-4-bromo-2-methylbut-2-en-1-al is acid sensitive, it would either decompose in the presence of the hydrobromic acid or further react therewith. Thus, it is readily seen that the prior art procedure disclose for preparation of the chlorinated derivatives is not suitable for the preparation of the corresponding brominated derivative and a different process or process conditions are required to obtain (E)-4-bromo-2-methylbut-2-en-1-al and to obtain good yields of said product.

It has also been known that 4-bromo-2-methylbut-2-en-1-al can be obtained from a reaction mixture of hydrobromic acid, 1,1-dimethoxy-2-methylbut-3-en-2-ol, toluene, sodium bromide and cupric bromide, for example, as disclosed in Example 3 of U.S. Patent 3,940,445 issued February 24, 1976, to Werner Reif et al. and assigned to Badische Anilin- & Soda-Fabrik Aktiengesellschaft. However, the yield obtained from said reaction is not acceptable and a more productive method is highly desired. Also, the starting materials are extremely difficult to prepare and the reaction is not stereospecific.

It has now been discovered accordingly the present invention that (E)-4-bromo-2-methylbut-2-en-1-al can be prepared easily and in high yields by brominating isoprene epoxide, namely 3,4-epoxy-3-methyl-1-butene, with cupric bromide in the presence of lithium carbonate.

According to this invention, isoprene epoxide may be brominated with at least a stoichiometric proportion of anhydrous cupric bromide, that is, two mols cupric bromide per mol of epoxide. The liquid phase bromination may be carried out at a refluxing temperature of from about 80° to about 90°C and may be carried out in any suitable solvent, such as for example, in a chloroform-ethyl acetate mixture.

The reaction is carried out in the presence of lithium carbonate which is present in at least an equimolar amount with respect to the amount of epoxide. The lithium carbonate is considered to be an acid sink for any hydrobromic acid formed in the reaction as well as exerting a catalytic effect on the reaction.

Preferably, the reaction is conducted under an inert atmosphere, preferably nitrogen, and under atmospheric pressure conditions. Most preferably, the reaction will be carried out under atmospheric pressure under dry nitrogen and at a temperature not to exceed 90°C for about half an hour and with the reaction being conducted in a 1:1 chloroform-ethyl acetate mixture.

The following example is illustrative of the process of this invention but is not limiting thereof.

Example

In 200 ml of a one to one chloroform-ethyl acetate mixture there is combined under dry nitrogen 8.50 g (10.0 ml—0.10 mol) 3,4-epoxide-3-methyl-1-butene, 44.68 g (0.20 mol) cupric bromide and 7.39 g (0.10 mol) lithium carbonate. The reaction mixture is heated to reflux for 35 minutes using a water bath that does not exceed 90°C. The reaction mixture is then cooled in ice bath to below ambient temperature or below about 20°C. About 100 ml of distilled water is added and the reaction mixture is stirred under dry nitrogen for about one hour and then the hydrolyzed reaction mixture is vacuum filtered under about 80 millimeters of mercury to remove the precipitated cuprous bromide.

The organic phase of the filtrate is separated from the aqueous phase and the aqueous phase is then extracted with about 150 to 200 mls of hexane. The organic phases are then combined and vacuum filtered under about 80 millimeters mercury to remove cuprous bromide and cupric bromide and then are washed with three 200 ml portions of water. After drying over anhydrous magnesium sulfate, the product is treated with about 2 g of activated charcoal (NUCHAR C-190N of J. T. Baker Chemical Co.) to decolorize the product by removal of organic impurities. After gravity filtration, the resulting filtrate is distilled at 40°C under a water aspirator vacuum to remove any solvents. The (E)-4-bromo-2-methylbut-2-en-1-al remains behind as a yellow-brown oil in a yield of 78% product having a purity of about 80%.

## Claims

1. A process for the production of (E)-4-bromo-2-methylbut-2-en-1-al comprising reacting 3,4-epoxy-3-methyl-1-butene with cupric bromide in the presence of lithium carbonate.

2. The process of claim 1 wherein the reaction is conducted at a reflux temperature of from about 70° to 90°C and in a chloroform-ethyl acetate solvent mixture.

## Patentansprüche

1. Ein Verfahren zur Herstellung von (E)-4-Brom-2-methyl-but-2-en-1-al, das die Umsetzung von 3,4-Epoxy-3-methyl-1-buten mit Kupfer(II)-bromid in Gegenwart von Lithiumcarbonat umfaßt.

2. Das Verfahren nach Anspruch 1, wobei die Umsetzung bei einer Rückflußtemperatur von etwa 70 bis 90°C und in einem Chloroform-Äthylacetat-Lösungsmittelgemisch durchgeführt wird.

## Revendications

1. Procédé de production de (E)-4-bromo-2-méthylbut-2-ène-1-al, comprenant la mise en réaction de 3,4-époxy-3-méthyl-1-butène avec du bromure cuivrique en présence de carbonate de lithium.

2. Procédé suivant la revendication 1, dans lequel on réalise la réaction à une température de reflux d'environ 70° à 90°C et dans un solvant formé d'un mélange de chloroforme et d'acétate d'éthyle.